# EUROPÄISCHE PATENTSCHRIFT

(11) **EP 0 561 122 B1**
(45) Date of publication and mention of the grant of the patent: **23.08.1995**
(21) Application number: 93101171.2
(22) Date of filing: 27.01.1993
(51) Int. Cl.: A61M 5/145

(54) **Pressure infusion apparatus**
Druckinfusionsapparat
Dispositif de perfusion par pression

(30) Priority: 17.03.1992 DE 19924208483
(43) Date of publication of application: 22.09.1993
(73) Proprietor: B. Braun Melsungen AG, D-34209 Melsungen (DE)
(72) Inventor: Gerlach, Hans Josef, W-3538 Marsberg (DE); Steger, Jürgen, W-3582 Felsberg (DE)
(74) Representative: Selting, Günther, Dipl.-Ing., et al

(56) References cited:
- WO-8 906 145
- EP-0 314 880
- EP-0 323 321
- EP-0 514 907

## Description

Die Erfindung betrifft einen Druckinfusionsapparat zum Injizieren von Medikamenten oder anderen Flüssigkeiten in den Körper eines Patienten.

Aus DE 37 37 331 C1 ist ein Druckinfusionsapparat bekannt, der einen an einem Gehäuse fest angebrachten ersten Spritzenhalter aufweist, an dem der Zylinder einer Infusionsspritze festgelegt wird. Die Kolbenstange der Spritze wird an einem linear bewegbaren zweiten Halter festgelegt, der von einem Antrieb in Richtung auf den ersten Halter bewegt wird. Der zweite Halter enthält einen Spritzendetektor, der die Anwesenheit einer an der Kolbenstange vorgesehenen Betätigungsplatte in dem zweiten Halter erkennt und die motorische Bewegung des zweiten Halters nur dann zuläßt, wenn eine Spritze detektiert wurde. Bei diesem Druckinfusionsapparat erfolgt der Antrieb des zweiten Halters über ein Schneckengetriebe, wobei eine Kupplung vorgesehen ist, um den zweiten Halter von dem Antrieb zu entkoppeln, damit nach dem Einlegen der Spritze der zweite Halter manuell in eine Stellung verschoben werden kann, die der Position der Betätigungsplatte der Spritze entspricht. Das Einlegen der Spritze und das Vorbereiten des Druckinfusionsapparates erfordern einen erheblichen manuellen Aufwand und zahlreiche Eingriffe. Zunächst muß der Antrieb entriegelt werden. Dann muß der Spritzenzylinder in den ersten Halter eingelegt werden. Schließlich muß der zweite Halter manuell auf die Stellung der Betätigungsplatte bewegt und die Betätigungsplatte in den zweiten Halter eingelegt werden. Dabei kann es vorkommen, daß eine ungenaue Positionierung des zweiten Halters erfolgt. Bei ungenauer Positionierung besteht die Gefahr, daß beim Einsetzen der Spritze der Kolben zurückgezogen und Luft angesaugt wird bzw. daß der Kolben vorgeschoben und wertvolles Medikament aus der Spritze ausgedrückt wird. Nachteilig ist ferner, daß zum Loskoppeln des Antriebes aufwendige mechanische Elemente, wie Kupplungen und Bedienelemente, erforderlich sind. Ferner tritt beim Einkuppeln des Antriebs ein mechanisches Spiel auf, das den Infusionsbeginn verzögert.

Von anderen Druckinfusionsapparaten ist bekannt, daß der zweite Halter mit Haltekrallen versehen ist. Diese sollen die Betätigungsplatte (Kolbenandruckplatte) halten. Die Stellung der Haltekrallen signalisiert dem Druckinfusionsapparat, daß die Spritze im zweiten Halter liegt. Der zusätzliche Nachteil dieser Anordnung liegt darin, daß die Haltekrallen nicht das wirkliche Vorhandensein der Betätigungsplatte erkennen. Ein falsches Einlegen der Spritze ist leicht möglich.

Ein Druckinfusionsapparat, von dem der Oberbegriff des Patentanspruchs 1 ausgeht, ist bekannt aus WO-A-8906145. Dieser Druckinfusionsapparat weist einen ersten Halter auf, an dem der Spritzenzylinder einer Spritze befestigt wird, und einen durch einen Antrieb linear bewegbaren zweiten Halter, der an einer Betätigungsplatte angreift, die am rückwärtigen Ende der Kolbenstange der Spritze vorgesehen ist. Durch Vorschieben des zweiten Halters wird der Spritzenkolben im Innern des Spritzenzylinders vorgeschoben und der Inhalt der Spritze ausgedrückt. Der zweite Halter ist mit einem Sensor versehen, der feststellt, wann der zweite Halter die Betätigungsplatte der Spritze erreicht. Beim Einsetzen einer Spritze in den ersten Halter ist der zweite Halter zurückgezogen. Dann wird durch Drücken eines Knopfes manuell ein Suchlauf eingeleitet, bei dem der zweite Halter schnell vorgeschoben wird, bis er die Betätigungsplatte erreicht. Wenn dies der Fall ist, erfolgt anschließend das weitere Vorschieben des zweiten Halters mit einer Geschwindigkeit, die der eingestellten Infusionsrate entspricht. Bei dieser Vorrichtung kann der Fall auftreten, daß der Suchlauf eingeleitet wird, während in den ersten Halter entweder ein Spritzenzylinder überhaupt nicht eingelegt ist, oder daß der Spritzenzylinder nicht ordnungsgemäß eingesetzt ist. Die Folge ist, daß der Druck im Infusionsapparat zwar seinen Betrieb ausführt, daß aber entweder keine Infusion erfolgt oder die Infusion nicht nach den vorgegebenen und erwarteten Kriterien erfolgt.

Der Erfindung liegt die Aufgabe zugrunde, einen Druckinfusionsapparat zu schaffen, der ein hohes Maß an Betriebssicherheit gewährleistet.

Die Lösung dieser Aufgabe erfolgt erfindungsgemäß mit den im Patentanspruch 1 angegebenen Merkmalen.

Bei dem erfindungsgemäßen Druckinfusionsapparat wird die Durchführung des Suchlaufs und der anschließende Infusionsvorgang nur dann ermöglicht, wenn der Spritzendetektor das Vorhandensein eines Spritzenzylinders in ordnungsgemäßer Lage am ersten Halter festgestellt hat. Dadurch wird die Funktionssicherheit des Druckinfusionsapparates verbessert.

Dabei ist der Antrieb des Druckinfusionsapparats über eine Steuervorrichtung derart gesteuert, daß er, wenn der am zweiten Halter vorgesehene Sensor das Vorhandensein einer Betätigungsplatte nicht feststellt, den zweiten Halter aus einer rückwärtigen Stellung heraus vorbewegt, bis der Sensor anspricht. Ein wesentlicher Vorteil besteht darin, daß die Spritze nur in den ersten Halter eingesetzt werden muß und daß der zweite Halter durch Motorantrieb an die gewissermaßen in Wartestellung befindliche Betätigungsplatte der Kolbenstange herangeführt wird, bis diese den Sensor betätigt hat. Die Spritze braucht also lediglich mit dem Spritzenzylinder am ersten Halter des Druckinfusionsapparates festgelegt zu werden. Danach wird das Gerät eingeschaltet und der zweite Halter fährt an die Betätigungsplatte der Spritze heran, bis der Sensor angesprochen hat. Dann kann der Infusionsvorgang beginnen. Vorteilhaft ist weiterhin, daß der Antrieb wesentlich vereinfacht ist, weil er keine Kupplungen oder Ausrast- und Bedienungselemente benötigt. Dadurch wird der mechanische Aufwand reduziert und die Herstellungskosten werden verringert. Außerdem wird vermieden, daß der Kolben der Spritze vor Infusionsbeginn verschoben wird, so daß keine Luft in die Spritze eingesaugt und keine Flüssigkeit vorzeitig aus der Spritze herausgedrückt wird.

Gemäß einer bevorzugten Ausgestaltung der Erfindung ist an dem zweiten Halter eine Greifvorrichtung vorgesehen, die nach Ansprechen des Sensors die Betätigungsplatte der Kolbenstange ergreift und damit eine sichere Fixierung der Kolbenstange am zweiten Halter bewirkt.

Im folgenden wird unter Bezugnahme auf die Zeichnungen ein Ausführungsbeispiel der Erfindung näher erläutert.

Es zeigen:

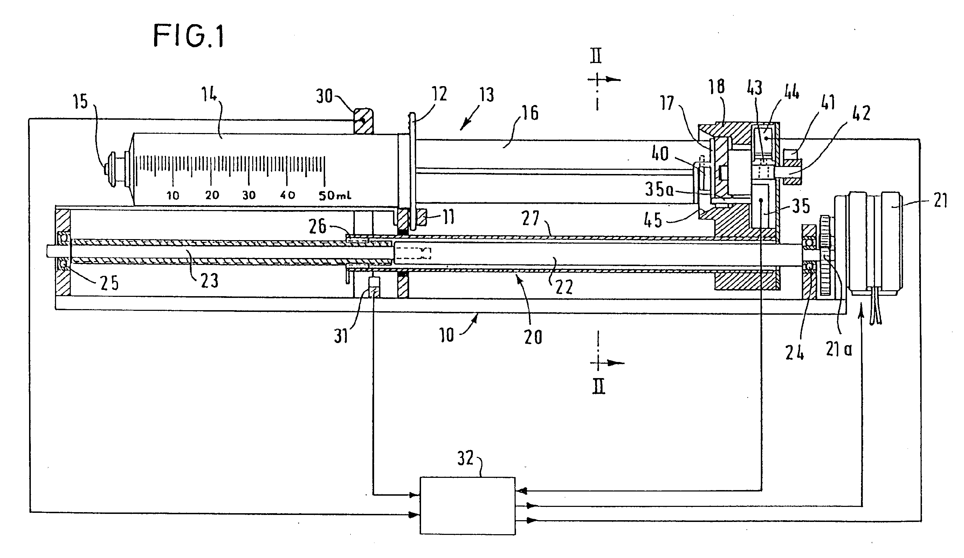

- Fig. 1: eine Draufsicht des Druckinfusionsapparates, teilweise geschnitten, mit eingelegter Spritze, und
- Fig. 2: einen Schnitt entlang der Linie II-II von Fig. 1, jedoch ohne eingelegte Spritze.

Der Druckinfusionsapparat weist ein Gehäuse 10 auf, das in der Zeichnung durch einen horizontalen Balken angedeutet ist, an dem die einzelnen Komponenten befestigt sind. Am Gehäuse 10 ist ein erster Halter 11 angeordnet, an dem die Halteplatte 12 einer Spritze 13 festgelegt werden kann. Die Halteplatte 12 ist am rückwärtigen Ende des Spritzenzylinders 14 angebracht, der an seinem vorderen Ende einen Auslaßstutzen 15 für den Anschluß einer zum Patienten führenden Flüssigkeitsleitung aufweist. Der erste Halter 11 hält die Spritze 13 in definierter axialer Ausrichtung fest. In dem Spritzenzylinder 14 befindet sich der (nicht dargestellte) Spritzenkolben, der mit einer Kolbenstange 16 verbunden ist, welche aus dem rückwärtigen Ende des Spritzenzylinders herausragt. Am rückwärtigen Ende der Kolbenstange 16 ist eine querverlaufende Betätigungsplatte 17 vorgesehen.

Die Betätigungsplatte 17 ist beim Betrieb des Druckinfusionsapparats in den zweiten Halter 18 eingesetzt, der linear in Richtung auf den ersten Halter bewegt werden kann, um den Spritzenkolben im Spritzenzylinder 14 vorzuschieben und die Flüssigkeit aus dem Auslaßstutzen 15 herauszudrükken.

Der zweite Halter 18 wird von einem Antrieb 20 angetrieben. Dieser Antrieb weist einen am Gehäuse 10 befestigten Motor 21 auf, dessen Ausgangswelle 22 mit einer Schraubenspindel 23 fest verbunden ist. Die Ausgangswelle 22 und die Schraubenspindel 23 sind in Lagern 24,25 im Gehäuse gelagert. Auf dem Schraubengewinde der Schraubenspindel 23 sitzt eine Spindelmutter 26, die über ein die Ausgangswelle 22 umgebendes Rohr 27 mit dem zweiten Halter 18 fest verbunden ist. Der Motor 21 treibt über ein Untersetzungsgetriebe 21a a die Ausgangswelle 22 an, wodurch die Schraubenspindel 23 gedreht wird. Dadurch wird die Spindelmutter 26, die gegen Drehung festgehalten ist, entlang der Schraubenspindel 23 linear bewegt. Diese Linearbewegung wird durch das Rohr 27 auf den zweiten Halter 18 übertragen.

An dem Gehäuse ist ferner ein Spritzendetektor 30 vorgesehen, der das Vorhandensein des Spritzenzylinders 14 in ordnungsgemäßer Lage an dem ersten Halter 11 erkennt. Ein weiterer Detektor 31 ist im Transportweg der Spindelmutter 26 angeordnet, um die Endstellung der Spindelmutter zu erkennen, wenn sich der zweite Halter 18 in seiner rückwärtigen Endposition befindet. Die Detektoren 30 und 31 liefern Signale an eine Steuervorrichtung 32, die hier als Mikroprozessor ausgebildet ist und die ihrerseits den Motor 21 steuert.

Der zweite Halter 18 enthält einen Sensor 35, der das Vorhandensein der Betätigungsplatte 17 in dem zweiten Halter erkennt. Der Sensor 35 ist ein Schalter mit einem Taststift 35a, welcher durch die Betätigungsplatte 17 zurückgedrückt wird, wenn diese sich im ersten Halter 18 befindet. Das Signal des Sensors 35 wird ebenfalls der Steuervorrichtung 32 zugeführt.

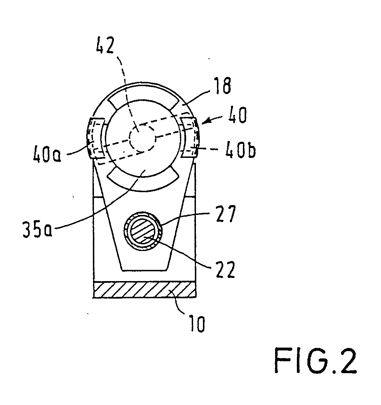

Der zweite Halter 18 weist ferner eine Greifvorrichtung 40 auf, mit zwei radial nach entgegengesetzten Seiten abstehenden Halteklauen 40a,40b (Fig. 2), die den Rand der Betätigungsplatte 17 übergreifen können. Der Rand der Betätigungsplatte 17 hat Aussparungen, die von den Halteklauen 40a,40b passiert werden können. Bei anschließender Drehung der Greifvorrichtung 40 um ihre Achse umgreifen die Halteklauen 40a und 40b die Betätigungsplatte und verriegeln diese an dem zweiten Halter 18. Die Greifvorrichtung 40 ist somit zwischen einem Freigabezustand und einem Greifzustand umschaltbar. Um die Greifvorrichtung in den Freigabezustand zu versetzen, wird ein Hebel 41 manuell betätigt, der auf derselben Achse 42 sitzt, an der auch die Halteklauen 40a,40b befestigt sind. Eine (nicht dargestellte) Feder übt eine Vorspannung auf die Achse 42 in dem Sinne aus, daß die Greifvorrichtung 40 in den Greifzustand versetzt wird. Bei Betätigung des Hebels 41 wird die Kraft der Feder überwunden und die Feder gespannt. Im Freigabezustand wird die Greifvorrichtung 40 durch einen Sperrbolzen 43 festgehalten. Dieser Sperrbolzen kann durch ein von der Steuervorrichtung 32 gesteuertes Betätigungselement 44, z.B. einen Elektromagneten, derart betätigt werden, daß er die Verriegelung des Freigabezustandes der Greifvorrichtung 40 aufgibt, wobei die Greifvorrichtung unter der Wirkung der Feder in den Greifzustand versetzt wird.

Der Druckinfusionsapparat arbeitet wie folgt: Durch manuelle Betätigung des Hebels 41 wird die Greifvorrichtung 40 in den Freigabezustand versetzt. Über den Sensor 42a wird der Freigabezustand erkannt und an die Steuervorrichtung signalisiert. Die Steuervorrichtung 32 steuert den Motor 21 so, daß der Halter 18 in seine rückwärtige Endposition gefahren wird, die von dem Detektor 31 erkannt wird. Wenn danach eine Spritze 13 in den ersten Halter 11 eingesetzt wird, wird dies von dem Spritzendetektor 30 erkannt und der Motor 21 wird in Funktion gesetzt, um den zweiten Halter 18 vorzutreiben. Am vorderen Ende des zweiten Halters 18 befindet sich eine Vertiefung mit konischem Rand 45, um die Betätigungsplatte 17, die am Ende der aus dem Spritzenzylinder 14 herausragenden Kolbenstange 16 angeordnet ist, zentrisch in den zweiten Halter 18 einzuführen. Der zweite Halter 18 fängt also bei seiner Vorwartsbewegung die Betätigungsplatte 17 und bringt sie in eine exakte axiale Ausrichtung zum Spritzenzylinder 14. Wenn die Betätigungsplatte 17 gegen den Taststift 35a stößt, gibt der Sensor 35 ein Signal an die Steuervorrichtung 32, wodurch der in einem Schnellgang bewirkte Vorschub des Halters 18 abgeschaltet wird. Anschließend erfolgt der Infusionsvorgang, bei dem der Motor 21 mit einer der vorgesehenen Infusionsrate entsprechenden relativ langsamen Geschwindigkeit läuft.

Auf das Signal des Sensors 35 hin bewirkt die Steuervorrichtung 32 die Erregung des Betätigungsorgans 44, das dann den Sperrbolzen 43 zurückzieht, so daß die Achse 42 sich unter der Wirkung ihrer Vorspannung drehen kann und die Greifvorrichtung 40 in den Greifzustand versetzt, in dem die Halteklauen 40a und 40b die Betätigungsplatte 17 übergreifen und festhalten. Der Zustand des Sperrbolzens 43 kann von einem zusätzlichen Detektor ermittelt und an die Steuervorrichtung 32 signalisiert werden.

Nach Beendigung des Infusionsvorganges kann die Spritze aus dem Gerät herausgenommen und durch eine neue Spritze ersetzt werden. Dabei wird der Hebel 41 manuell betätigt, um die Greifvorrichtung in den Freigabezustand zu versetzen, in dem sie durch den Sperrbolzen 43 verriegelt wird. Bevor eine neue Spritze eingesetzt wird, wird der Halter 18 in seine rückwärtige Endstellung fahren.

Abweichend von dem beschriebenen Ausführungsbeispiel, bei dem die Betätigung der Greifvorrichtung 40 über den Hebel 41 und das Betätigungsorgan 44 erfolgt, ist es auch möglich, die Greifvorrichtung über einen Getriebemotor zu betätigen. Das Öffnen der Greifvorrichtung kann manuell über ein Bedienelement, z.B. eine Taste, ausgelöst werden. Bei Erkennung der Betätigungsplatte 17 durch den Sensor wird die Greifvorrichtung durch den Motor geschlossen.

Eine weitere Möglichkeit sieht vor, daß die Greifvorrichtung nach Aktivierung des Sensors einen zweiten Getriebemotor bewegt wird.

## Claims

1. A pressure infusion apparatus, comprising a first holder (11) for fixing the barrel (14) of a syringe (13), a second holder (18) for engaging an actuator plate (17) at the rear end of the piston rod (16) of the syringe (13), a drive means (20) for linear movement of the second holder (18) relative to the first holder (11), and a sensor (35) provided at the second holder (18) for detecting the presence of the actuator plate (17), the drive means (20) being controlled by a control means (32) to advance the second holder (18) in a search run out of a rearward position until the second holder reaches the actuator plate (17) and then excites the sensor (35) and terminates the search run,
**characterized in**
**that** a syringe detector (30) is provided to detect the presence of a syringe barrel (14) in a correct position at the first holder (11), the syringe detector (30) allowing activation of the drive means (20) for the search run of the second holder (18) only when detecting a syringe.

2. The pressure infusion apparatus according to claim 1, **characterized in that** the second holder (18) is provided with a gripping means (40) switchable between a release state and a gripping state and controlled by the sensor (35) to be set into the gripping state and hold the actuator plate (17) when the actuator plate is detected.

3. The pressure infusion apparatus according to claim 1 or 2, **characterized in that** the control means (32) deactivates the drive means in response to the signal of the sensor (35).

4. The pressure infusion apparatus according to any one of claims 1 to 3, **characterized in that** a hand-operated unlocking device (41) is provided to set the gripping means (40) to the release state.

5. The pressure infusion apparatus according to any one of claims 2 to 4, **characterized in that** the gripping means (40) is spring-biased and provided with a locking member (43) for maintaining the release state, said locking member (43) having associated thereto an actuating member (44) which can be unlocked by the detection signal of the sensor (35).

6. The pressure infusion apparatus according to claim 5, **characterized in that** the actuating member (44) is arranged as an electromechanical actuating member (44).

## Patentansprüche

1. Druckinfusionsapparat mit einem ersten Halter (11) zum Festlegen des Zylinders (14) einer Spritze (13), einem zweiten Halber (18) zum Angreifen an einer Betätigungsplatte (17) am rückwärtigen Ende der Kolbenstange (16) der Spritze (13), einem Antrieb (20) zum linearen Bewegen des zweiten Halters (18) relativ zu dem ersten Halber (11) und einem an dem zweiten Halber (18) vorgesehenen Sensor (35) zum Erkennen des Vorhandenseins der Betätigungsplatte (17), wobei der Antrieb (20) durch eine Steuervorrichtung (32) derart gesteuert ist, daß er in einem Suchlauf den zweiten Halter (18) aus einer rückwärtigen Stellung heraus vorbewegt, bis der zweite Halber die Betätigungsplatte (17) erreicht und daraufhin der Sensor (35) anspricht und den Suchlauf beendet,
**dadurch gekennzeichnet,**
**daß** ein das Vorhandensein eines Spritzenzylinders (14) in ordnungsgemäßer Lage am ersten Halter (11) feststellender Spritzendetektor (30) vorgesehen ist, der die Aktivierung des Antriebs (20) für den Suchlauf des zweiten Halters (18) nur dann zuläßt, wenn er eine Spritze erkennt.

2. Druckinfusionapparat nach Anspruch 1, **dadurch gekennzeichnet, daß** der zweite Halter (18) eine Greifvorrichtung (40) aufweist, die zwischen einem Freigabeszustand und einem Greifzustand umschaltbar ist und die von dem Sensor (35) derart gesteuert ist, daß sie bei Erkennen der Betätigungsplatte in den Greifzustand versetzt wird und die Betätigungsplatte (17) festhält.

3. Druckinfusionsapparat nach Anspruch 1 oder 2, **dadurch gekennzeichnet, daß** die Steuervorrichtung (32) auf das Signal des Sensors (35) hin den Antrieb stillsetzt.

4. Druckinfusionsapparat nach einem der Ansprüche 1-3, **dadurch gekennzeichnet, daß** eine manuell betätigbare Entriegelungsvorrichtung (41) vorgesehen ist, um die Greifvorrichtung (40) in den Freigabezustand zu versetzen.

5. Druckinfusionsapparat nach einem der Ansprüche 2-4, **dadurch gekennzeichnet, daß** die Greifvorrichtung (40) federgespannt ist und ein Sperrelement (43) zum Beibehalten des Freigabezustandes aufweist, wobei dem Sperrelement (43) ein Betätigungselement (44) zugeordnet ist, das durch das Erkennungssignal des Sensors (35) entriegelbar ist.

6. Druckinfusionsapparat nach Anspruch 5, **dadurch gekennzeichnet, daß** das Betätigungselement (44) als elektromechanisches Betätigungselement (44) ausgebildet ist.

## Revendications

1. Appareil de perfusion par pression, comportant un premier élément de maintien (11) pour la fixation du cylindre (14) d'une seringue (13), un deuxième élément de maintien (18) destiné à saisir une plaque d'actionnement (17) à l'extrémité arrière de la tige (16) du piston de la seringue (13), un dispositif d'entraînement (20) pour mouvoir linéairement le deuxième élément de maintien (18) par rapport au premier élément de maintien (11) et un capteur (35) prévu au deuxième élément de maintien (18) pour la reconnaissance de la présence de la plaque d'actionnement (17), le dispositif d'entraînement (20) étant commandé par un dispositif de commande (32) d'une façon telle que, dans une course de recherche, il meut le deuxième élément de maintien (18) vers l'avant, à partir d'une position arrière, jusqu'à ce que le deuxième élément de maintien atteigne la plaque d'actionnement (17), à la suite de quoi le capteur (35) réagit et met fin à la course de recherche,
**caractérisé en ce,**
**qu'**il est prévu un détecteur de seringue (30) déterminant la présence d'un cylindre de seringue (14) dans une position réglementaire au premier élément de maintien (11), détecteur qui n'autorise l'activation du dispositif d'entraînement (20) pour la course de recherche du deuxième élément de maintien (18), que lorsqu'il reconnaît une seringue.

2. Appareil de perfusion par pression selon la revendication 1, **caractérisé en ce que** le deuxième élément de maintien (18) présente un dispositif de prise (40), qui peut être commuté entre un état de libération et un état de prise et qui est commandé par le capteur (35) d'une façon telle que, lorsque la plaque d'actionnement est reconnue, il soit décalé dans l'état de prise et retienne la plaque d'actionnement (17).

3. Appareil de perfusion par pression selon la revendication 1 ou 2, **caractérisé en ce que** le dispositif de commande (32) met le dispositif d'entraînement à l'arrêt sur le signal du capteur (35).

4. Appareil de perfusion par pression selon l'une des revendications 1 à 3, **caractérisé en ce qu'**un dispositif de déverrouillage (41) à actionnement manuel est prévu, pour décaler le dispositif de prise (40) dans l'état de libération.

5. Appareil de perfusion par pression selon l'une des revendications 2 à 4, **caractérisé en ce que** le dispositif de prise (40) est contraint par un ressort et présente un élément de blocage (43) pour maintenir l'état de libération, l'élément de blocage (43) étant associé à un élément d'actionnement (44), qui peut être déverrouillé par le signal de reconnaissance du capteur (35).

6. Appareil de perfusion par pression selon la revendication 5, **caractérisé en ce que** l'élément d'actionnement (44) est constitué sous la forme d'un élément d'actionnement électromécanique (44).
